(19) Europäisches Patentamt
European Patent Office
Office européen des brevets

(11) Veröffentlichungsnummer: **0 451 560 A2**

# (12) EUROPÄISCHE PATENTANMELDUNG

(21) Anmeldenummer: **91104293.5**

(51) Int. Cl.⁵: **A61B 5/083**, A61B 5/00

(22) Anmeldetag: **20.03.91**

(30) Priorität: **05.04.90 DE 4011065**

(43) Veröffentlichungstag der Anmeldung:
**16.10.91 Patentblatt 91/42**

(84) Benannte Vertragsstaaten:
**CH DE FR GB IT LI**

(71) Anmelder: **PPG Hellige GmbH**
**Heinrich-von-Stephan-Str. 4**
**W-7800 Freiburg(DE)**

(72) Erfinder: **Kronberg, Harald, Dr.Dipl.-Phys.**
**Mülhauser Strasse 6**
**W-7813 Staufen(DE)**
Erfinder: **Leist, Helmut, Dipl.-Ing.**
**Tannenweg 45a**
**W-7808 Waldirch(DE)**

## (54) Vorrichtung zur Atmungsüberwachung.

(57) Die Vorrichtung zur Atmungsüberwachung umfaßt einen photoplethysmographischen Sensor (1), dessen LED-Senderelement(e) (5) von einem Überwachungsgerät (20) mit einer bestimmten Ansteuerleistung und/oder Tastsignalfolge bzw. Frequenz erregt werden. Durch eine Signalverarbeitungseinheit (9 bis 15) lassen sich sowohl die Atemfrequenz und Atemtiefe als auch die Pulsfrequenz bzw. Sauerstoffsättigung des peripheren Bluts erfassen. Damit ist eine wesentlich zuverlässigere Atmungsüberwachung gegenüber der üblichen impedanzpneumographischen Methode gewährleistet.

Fig.7

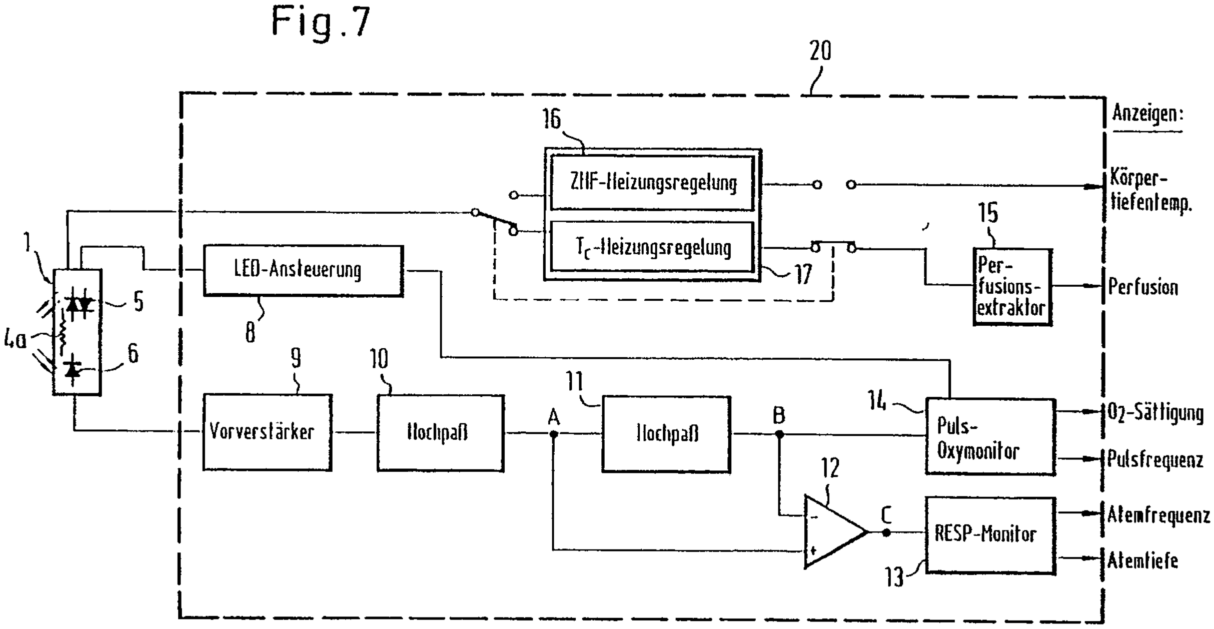


EP 0 451 560 A2

Die Erfindung betrifft eine Vorrichtung zur Atmungsüberwachung durch periphere Erfassung von atmungsrelevanten Vitalmeßgrößen.

Die meßtechnische Erfassung der Atmung gehört zu den Grundanforderungen intensivmedizinischer Überwachung und Diagnostik. Während sehr befriedigende Meßmethoden existieren für den intubierten oder eine Atemmaske tragenden Patienten, sind die Probleme bei nichtinvasiver Atemüberwachung hinsichtlich klinischer Handhabung, Zuverlässigkeit, Genauigkeit und Reproduzierbarkeit der Meßergebnisse bis heute nicht befriedigend gelöst. Atemsensoren auf Thermistorbasis im Atemstrom vor Mund und/oder Nase sind schwierig zu applizieren und werden häufig vom Patienten nicht toleriert. Atemgürtel um Brust und Bauch zur mechanischen Lungenplethysmographie sind pflegeintensiv und erfordern zusätzliche Patientenleitungen und -überwachungsgeräte (vgl. UK-B 2 116 725).

Es ist bekannt, daß während der Atmung synchrone periodische Schwankungen der Blutfülle in allen Körperkompartimenten auftreten, teilweise als Folge einer oxygenationssynchronen Blutdruckmodulation, vor allem aber durch mechanische Stau- und Pumpwirkung. So steigt beispielsweise der Hirndruck während der Expiration wegen venösen Blutrückstaus (vgl. Lit. 1). Ausgeprägter ist die Verdrängung des Bluts auf der Thoraxoberfläche während der Inspiration. Aus diesem Grund hat sich zur Zeit die Impedanzpneumographie zur Atemüberwachung durchgesetzt, bei der über EKG-Brustelektroden eine atemabhängige Modulation der Thoraximpedanz um etwa 1 ‰ gemessen wird (vgl. Lit. 2). Leider wird dieses schwache Atemsignal leicht gestört durch Artefakte, eine körperlageabhängige Basisliniendrift sowie bei der üblichen Zweileitermeßtechnik durch lokale Elektroden-Haut-Übergangsimpedanzen. Zwar erfordert die Impedanzpneumographie bei Zweileitermeßtechnik neben der EKG-Registrierung kaum erhöhten Handhabungsaufwand, doch ist meist eine befriedigende Atemüberwachung nur bei höherem Pflegeaufwand mit zusätzlich geklebten Elektroden in Vierleitermeßtechnik zu erreichen. Ein grundsätzlicher Nachteil dieser Meßmethode liegt darin, daß eine meßbare Atembewegung keineswegs ein Zeichen effektiver Atmung sein muß, so nur beispielshalber bei Atemwegsobstruktion oder unkoordinierter phasenverschobener Brust- und Bauchatmung bei Säuglingen mit unreifem Atmungssystem.

Der Erfindung liegt die Aufgabe zugrunde, die nichtinvasive Atmungsüberwachung hinsichtlich klinischer Handhabbarkeit, Zuverlässigkeit und Reproduzierbarkeit des Meßergebnisses zu verbessern.

Mit der Erfindung wird eine Vorrichtung zur Atmungsüberwachung durch periphere Erfassung von atmungsrelevanten Vitalmeßgrößen bereitgestellt, die erfindungsgemäß gekennzeichnet ist durch

- einen auf die Oberfläche eines von Blutgefäßen durchsetzten Organs, vorzugsweise im Bereich des Oberkörpers eines Probanden zu applizierenden Sensor, der
  -- einen Strahlungssender, der elektromagnetische Strahlung in das Organgewebe emittiert und
  -- einen Strahlungsempfänger enthält, der nach Wechselwirkung der Strahlung mit dem Organgewebe einen Strahlungsanteil aufnimmt,
- eine Ansteuereinrichtung, die den Strahlungssender mit einer gewünschten Ansteuerleistung, Tastsignalfolge und Tastsignalfolgefrequenz erregt und durch
- eine signalverarbeitungseinheit, welche das Ausgangssignal des Strahlungsempfängers aufnimmt und mindestens ein dem zeitlichen Verlauf der Atemexkursionen entsprechendes Ausgangssignal bereitstellt und/oder Atemfrequenz und/oder Atemtiefe bestimmt.

Gegenüber der bekannten impedanzpneumographischen Atmungsüberwachung hat die erfindungsgemäße Vorrichtung zur Atmungsüberwachung bereits in der einfachsten, nur die Atemfrequenz ermittelnden Ausführung, den großen Vorzug eines wesentlich stabileren und störfesteren Meßsignals.

Eine wesentliche Verbesserung der Atmungsüberwachung insbesondere bei obstruktiver oder unkoordinierter Atmung ergibt sich, wenn die oben definierte Atmungsüberwachungsvorrichtung zusätzlich mit einem bekannten Pulsoxymonitor (vgl. US-A-4 407 290) kombiniert wird. Neben der üblichen pulsoxymetrischen Erfassung von arterieller Sauerstoffsättigung und Pulsfrequenz wird dann die Atmung ohne zusätzlichen Handhabungsaufwand überwacht, so daß frustrane Atemexkursionen über den Abfall der Sauerstoffsättigung des Hämoglobins erkannt und alarmiert werden. In dieser Kombination wird der im Sensor enthaltene Strahlungssender auf bekannte Weise sequentiell bei mindestens zwei meist im sichtbaren und nahen Infrarotbereich liegenden Wellenlängen, z. B. 660 nm und 940 nm, erregt und sein Ausgangssignal wird zur Bestimmung der Sauerstoffsättigung und Pulsfrequenz in an sich bekannter Weise in der Signalverarbeitungseinheit ausgewertet. Verfahren zur photoplethysmographischen Erfassung der peripheren lokalen Durchblutung sind an sich in verschiedenen Ausführungsformen bekannt. Je nach Meßart werden hierfür Transmissions- oder Reflexionssensoren verwendet (vgl. beispielshalber US-A-3 152 587 und UK-B 987 504). Wenn zur Photoplethysmographie eine infrarote Strahlungsquelle mit für Hämoglobin und Oxyhämoglobin isosbestischer Wellenlänge von 805 nm verwendet wird, ist das Meßsignal unabhängig von der Sauerstoffsättigung des Bluts. Bislang wurden solche Aufnehmer jedoch nur zur Überwachung der Pulsfrequenz eingesetzt, während andere Signalkomponenten lediglich als Störung unterdrückt wurden.

Im Rahmen der Zweiwellenlängen-Pulsoxymetrie wurden aber auch Verfahren zur Quantifizierung der körperoberflächennahen Blutperfusion entwikkelt, die nicht mehr auf die Verwendung einer isosbestischen Wellenlänge angewiesen sind (vgl. EP-A-0 293 504). Damit läßt sich allerdings nur eine allmähliche Perfusionsänderung erfassen, wie z. B. im Verlauf von Operationen.

Bekannt ist es auch, ähnliche Perfusionsgrößen kalorimetrisch zu erfassen. Dazu sind auf der Haut fixierte transcutane Blutgassensoren geeignet, die mit einer Heizung zur Hyperämisierung des arteriellen Kapillarbetts ausgerüstet sind. Aus der für eine konstante hypertherme Hauttemperatur erforderlichen Heizleistung kann dann auf die Perfusionseffizienz und den Perfusionsdruck geschlossen werden (vgl. DE-A-22 55 879 bzw. DE-B-24 23 441).

Der Erfindung liegt unter anderem die Idee zugrunde, die bekannten Meßmethoden oder einzelne dadurch bekannt gewordene Meßsysteme auch zur Erfassung atemsynchroner Perfusionsänderungen heranzuziehen. Bislang wurden weder die photoplethysmographischen noch die kalorimetrischen Verfahren zur Atemüberwachung eingesetzt.

Die erfindungsgemäße Vorrichtung könnte auch als photoplethysmographisches Überwachungssystem bezeichnet werden, das vorteilhaft mit zusätzlichen Meßsonden oder -sensoren ausgerüstet sein kann zur gleichzeitigen Überwachung weiterer, häufig erforderlicher Vitalmeßgrößen, wie Blutgaspartialdruck des arteriellen Bluts, peripherer Blutperfusion und/oder Körpertiefentemperatur.

Die Erfindung und vorteilhafte Einzelheiten werden nachfolgend unter Bezug auf die Zeichnungen in beispielsweiser Ausführungsform näher erläutert. Es zeigen:

| | |
|---|---|
| Fig. 1 und 2 | photoplethysmographische Reflexsensoren, wie sie in Verbindung mit der Erfindung vorteilhaft eingesetzt werden können; |
| Fig. 3 | ein Diagramm für ein typisches Meßsignal eines Sensors gemäß den Fig. 1 oder 2 am Ausgang eines Vorverstärkers; |
| Fig. 4 | ein interessierendes Meßsignal, das drei Signalanteile enthält, nämlich Atemfrequenz, Atemtiefe und Pulsfrequenz; |
| Fig. 5 | das herausgefilterte Pulssignal; |
| Fig. 6 | das herausgefilterte Atmungssignal; und |
| Fig. 7 | das Prinzipblockschaltbild einer vollständigen Atmungsüberwachungsvorrichtung, bei der vorteilhafterweise noch zusätzliche Vitalmeßgrößen gewonnen und angezeigt werden können. |

Die Fig. 1 und 2 verdeutlichen beispielhaft zwei verschiedene Ausführungsformen photoplethysmographischer Reflexsensoren 1, die z. B. mit EKG-Elektrodenkleberingen 7 direkt auf eine Organoberfläche 3 geklebt werden können, vorteilhafterweise auf die Haut des Oberkörpers im Bereich des Brustbeins, der Brustmuskeln, zwischen oder auf den Schulterblättern. Leuchtdioden 5 (LEDs) dienen als Strahlungsquelle, wobei zwei oder mehr solcher LEDs zur zusätzlichen Erfassung vorbestimmter Blutbestandteile vorgesehen sein können. Der aus dem Gewebe empfangene modifizierte Strahlungsanteil wird durch Photodioden 6 (PHDs) als Empfänger aufgenommen, die mit bestgeeignetem, gegenseitigem Abstand von etwa 3 mm bis 15 mm innerhalb eines Heizkörpers 4 angeordnet sind, umgeben von einem Fixierring 2, der gleichzeitig zur Störstrahlungsabsorbierung dient. Der Heizkörper 4 ist z. B., wie angedeutet, über eine umlaufende Widerstandsdrahtwicklung 4a oder eingekittete Heizwiderstände beheizt.

Die Fig. 3 zeigt ein typisches infrarotes Meßsignal am Ausgang eines Vorverstärkers. Der Meßwert von ca. -1000 mV ist mit pulssynchronen (z. B. 72 Pulse/min) und atemsynchronen (z. B. 11 Atemzüge/min) Signalschwankungen moduliert. Die Amplitude des überlagerten Wechselspannungsanteils ist proportional zum Signalmittelwert, so daß für die weitere Signalverarbeitung nur auf diesen Mittelwert bezogene relative Wechselspannungen (Quotient aus Wechselspannungs- und Gleichspannungsanteil) interessieren. Die Amplitudenwerte liegen in der Größenordnung von 1 %, bezogen auf den Signalmittelwert der Fig. 3; sie sind damit wesentlich störsicherer als das Signal bei einer Thoraximpedanzmessung.

Zur photometrisch-technischen Optimierung der Signalverarbeitung kann der Wechselspannungsanteil auf die Signalspitzenwerte normiert werden, die sich aber, wie angegeben, normalerweise nur um etwa 1 % vom Mittelwert unterscheiden. Zur Unterdrückung von Artefakten ist es dabei sinnvoll, den Mittelwert des Meßsignals oder gemittelte Spitzenwerte über gleitende Zeitfenster zu bestimmen.

Im Rahmen der zur Erfindung gehörenden Untersuchungen wurde festgestellt, daß die relative Wechselspannung mit Erhöhung der Heiztemperatur zwischen 37° C und 44° C wächst, ebenso mit dem Abstand zwischen den Leuchtdioden 5 und den Photodioden 6.

Der Reflexsensor gemäß Fig. 2 läßt gegenüber der Ausführungsform nach Fig. 1 einen vergrößerten LED-PHD-Abstand erkennen bei asymmetrischer Anordnung der Sender- bzw. Empfängerelemente, jedoch unveränderter Gesamtgröße. Zur Signaloptimierung können außerdem die LEDs 5 und die PHDs 6 in einem gewissen Anstellwinkel zur Oberfläche des Gewebes 3 angeordnet sein, was sich aus der Schnittdarstellung im linken Teil der Fig. 2 erkennen läßt.

Die Fig. 7 zeigt beispielhaft das Funktionsschema bzw. das Blockschaltbild einer erfindungsgemäßen Überwachungsvorrichtung 20, an die der Sensor 1 angeschlosen wird. Nach Vorverstärkung und Impedanzwandlung im Vorverstärker 9 sowie Eliminierung einer langsamen Signaldrift in einem Hochpaß 10 wird am Punkt A das Signal gemäß Fig. 3 beobachtet, das noch alle Spektralkomponenten oberhalb von zehn Perioden/min enthält. Bei dem Meßsignal der Fig. 3 war ein Reflexsensor gemäß Fig. 2 auf das Brustbein eines selbständig atmenden Probanden aufgeklebt und das unterliegende Kapillarbett im Gewebe 3 war 10 Minuten lang bei 44°C hyperämisiert worden. Das Diagramm der Fig. 4 zeigt als interessierendes Meßsignal die am Punkt A der Schaltung nach Fig. 7 gewonnene relative, auf den Gleichspannungsanteil normierte Wechselspannung.

Zur Ermittlung aller aus dem arteriellen Puls ableitbaren Vitalmeßwerte (z. B. Pulsperfusion bei 805 nm-Einwellenlängenphotometrie, Pulsfrequenz, zusätzlich Sauerstoffsättigung des Hämoglobins bei mindestens Zweiwellenlängenphotometrie mittels eines Pulyoxymonitors 14 werden atembedingte tieffrequente Signaländerungen in einem weiteren Hochpaß 11 eliminiert.

Die Fig. 5 zeigt die relative Wechselspannung am Punkt B der Schaltungsanordnung nach Fig. 7, wenn alle Spektralkomponenten unterhalb von 36 Perioden/min des Signals am Punkt A (Fig. 4) ausgefiltert werden.

Am Punkt C, nämlich nach Verknüpfung der Signale an den Punkten A bzw. B über einen Differenzverstärker 12, erscheint am Eingang eines Respirationsmonitors 13 ein als Differenz der Spannungen in den Punkten A und B leicht auszuwertendes Atemsignal mit einer relativen Wechselspannung gemäß Fig. 6, aus dem die Atemfrequenz und Atemtiefe auf bekannte Weise, ähnlich wie bei der Impedanzpneumographie, gewonnen werden.

Das Funktionsschema der Fig. 7 zeigt zwei Sensor-Ansteueranschlüsse, und zwar einerseits für den Betrieb der LEDs 5 über eine dem Pulsoxymonitor 14 zugeordnete LED-Ansteuerungseinheit 8 und andererseits für die Sensorheizung über eine umschaltbare Heizungseinheit 16, 17. Wird der Sensor 1 zur Hyperämie über den Pulsoxymonitor 14 auf konstante Temperatur $T_c$ geheizt, so ist es möglich, daß der im dargestellten Ausführungsbeispiel ebenfalls angeschlossene Perfusionsextraktor 15 die lokale Organperfusion sowohl kalorimetrisch über die Heizungsregelung als auch photometrisch über den Meßsignalzweig 9-10-11-14 ermittelt, gegebenenfalls durch Vergleich sogar kontrollieren kann.

Möglich ist auch die Umschaltung auf eine alternative Sensorheizung 16, um zusätzlich die Körpertiefentemperatur zu erfassen nach einer Meßmethode, die als Zero-Heat-Flow-Verfahren vorgeschlagen worden ist (vgl. EP-Patentanmeldung Nr. 89 109 162.1).

Zur Pulsoxymetrie bei der Messung mit mindestens zwei Wellenlängen ist das Funktionsschema der Fig. 7 - für den Fachmann ersichtlich - im Meßzweig 9-10-11-14 mehrkanalig ausgeführt. Ist weiterhin der Reflexsensor (Fig. 2) mit zusätzlichen Sensorkomponenten kombiniert, z. B. mit Elektroden zur transcutanen Blutgasüberwachung, so enthält das Überwachungsgerät zusätzlich eine entsprechende Kombination von Funktionsschaltkreisen.

Die Angaben in Fig. 7 zu den Filtergrenzfrequenzen sind nicht starr aufzufassen. Zwar lassen sich Pulsfrequenz und Atemfrequenz fast immer einfach trennen, da sie meist im Verhältnis von 4:1 stehen. In vorteilhafter Ergänzung des Erfindungsgedankens ist es jedoch sinnvoll, die Grenzfrequenzen dem Alter des Probanden anzupassen, da z. B. bei Neugeborenen Puls- und Atemfrequenz etwa dreimal höher als bei Erwachsenen liegen.

Eine zusätzliche Möglichkeit, die Atmung aus der bekannten respiratorischen Pulsfrequenz-Arrhythmie zu ermitteln (bei Inspiration steigt die Pulsfrequenz) ist im Schema der erfindungsgemäßen Überwachungsvorrichtung nach Fig. 7 nicht ausgeführt. Dies wäre vorzugsweise mittels digitaler Signalauswertung und Mustererkennung zu realisieren.

## LITERATURREFERENZEN


Lit 1: ALLEN, R.; Intercranial pressure; a review of clinical problems, measurement techniques and monitoring methods. J. Med. Engng. + Techn. 10, 299-320 (1986)

Lit 2: ANDERSON Jr. F.A.: Impedance Plethysmography, in: WEBSTER, J.G. (ed.): Encyclopedia of Medical Devices and Instrumentation, Vol. 3, 1641-1643, John Wiley + Sons, 1988


## Patentansprüche

1. Vorrichtung zur Atmungsüberwachung durch periphere Erfassung von atmungsrelevanten Vitalmeßgrößen, **gekennzeichnet durch**

- einen auf die Oberfläche eines von Blutgefäßen durchsetzten Organs (3) vorzugsweise im Bereich des Oberkörpers eines Probanden zu applizierenden Sensor (1), der
  -- einen Strahlungssender (5), der elektromagnetische Strahlung in das Organgewebe emittiert und
  -- einen Strahlungsempfänger (6) enthält, der nach Wechselwirkung der elektromagnetischen Strahlung mit dem Organgewebe einen Strahlungsanteil aufnimmt,
- eine Ansteuereinrichtung (8, 16, 17), die den Strahlungssender (5) mit einer gewünschten Ansteuerleistung und/oder Tastsignalfolge erregt und durch
- eine Signalverarbeitungseinheit (9 bis 15), welche das Ausgangssignal des Strahlungsempfängers (6) aufnimmt und Meßwerte für die Atemaktivität des Probanden bestimmt.

**2.** Vorrichtung nach Anspruch 1, **dadurch gekennzeichnet,** daß

- der Strahlungssender (5) durch die Ansteuereinrichtung (8, 16, 17) zu elektromagnetischer Strahlung bei mindestens zwei unterschiedlichen Frequenzen (Wellenlängen) erregt wird,
- der Strahlungsempfänger (6) Ausgangssignale für die nach Wechselwirkung mit dem Organgewebe empfangenen mindestens zwei Strahlungsanteile liefert und
- eine Signalverarbeitungseinheit (9 bis 15) aus den Ausgangssignalen Meßwerte für die mechanische Atemaktivität, für vorbestimmte Blutbestanteile und/oder Blutkreislaufgrößen bestimmt.

**3.** Vorrichtung nach Anspruch 2, **dadurch gekennzeichnet,** daß das Signalverarbeitungsmittel (14) ein Pulsoxymonitor zur Bestimmung von mindestens der Sauerstoffsättigung des peripheren arteriellen Bluts im Organgewebe ist.

**4.** Vorrichtung nach Anspruch 2, **dadurch gekennzeichnet,** daß als Blutkreislaufgröße mindestens die Pulsfrequenz bestimmt wird.

**5.** Vorrichtung nach Anspruch 1 und 2, **dadurch gekennzeichnet,** daß die Signalverarbeitungsmittel (10 bis 13) den zeitlichen Verlauf der Atemexkursionen und/oder die Atemfrequenz und/oder die Atemtiefe bestimmen.

**6.** Vorrichtung nach Anspruch 1 oder 2, **dadurch gekennzeichnet,** daß die Signalverarbeitungsmittel (10 bis 13) einen Bandpaß (10, 11) mit festen, einstellbaren oder automatisch adaptierenden Grenzfrequenzen enthalten.

**7.** Vorrichtung nach Anspruch 6, **dadurch gekennzeichnet,** daß die Grenzfrequenzen des Bandpasses im Bereich von 8 Perioden/min $\leqq f_G \leqq$ 50 Perioden/min und etwa im Verhältnis 4:1 gewählt sind.

**8.** Vorrichtung nach Anspruch 1 bis 5, **dadurch gekennzeichnet,** daß die Signalverarbeitungseinheit (9 bis 15) mittels Hochpaß (10) eine allmähliche Signaldrift eliminiert.

**9.** Vorrichtung nach Anspruch 1 bis 5, **dadurch gekennzeichnet,** daß die Ausgangssignale des Vorverstärkers (9) auf deren Mittelwerte oder Spitzenwerte normiert sind.

**10.** Vorrichtung nach Anspruch 9, **dadurch gekennzeichnet,** daß die Mittelwerte oder Spitzenwerte über begrenzte Zeitabschnitte zeitgleitend ermittelt werden.

**11.** Vorrichtung nach Anspruch 1 und 2, **dadurch gekennzeichnet,** daß die Strahlungssender (5) Leuchtdioden vorbestimmter Strahlungsfrequenz (Strahlungswellenlänge) im sichtbaren und nahen Infrarotbereich sind.

**12.** Vorrichtung nach Anspruch 1 und 2, **dadurch gekennzeichnet,** daß eine Leuchtdiode als Strahlungssender (5) mit einer Strahlungsfrequenz im nahen Infrarotbereich, insbesondere bei 805 nm, vorgesehen ist.

**13.** Vorrichtung nach Anspruch 1, 2 bzw. 11 oder 12, **dadurch gekennzeichnet,** daß die Strahlungsempfänger (6) Photodioden mit auf die Strahlungsfrequenz(en) (Strahlungswellenlänge(n)) der (des) Strahlungssender(s) (5) angepaßte(r)n spektralte(r)n Empfindlichkeit(en) ist (sind).

**14.** Vorrichtung nach einem der vorstehenden Ansprüche, **dadurch gekennzeichnet,** daß der Sensor (1) mit einer Einrichtung (4, 4a) zur Beheizung und die Signalverarbeitungseinheit (20) mit einer Einrichtung zur Thermostatisierung (17) des Sensors auf Hyperämisierungstemperatur des angrenzenden Körpergewebes (3) ausgerüstet ist.

**15.** Vorrichtung nach Anspruch 14, **dadurch gekennzeichnet,** daß das Signalverarbeitungsgerät (20) mit einer Einrichtung (15) zur Anzeige eines von der Heizleisstung abgeleiteten Maßes für die Blutperfusion ausgerüstet ist.

**16.** Vorrichtung nach einem der vorstehenden Ansprüche 1 bis 13, **dadurch gekennzeichnet,** daß der Sensor (1) mit einer Einrichtung (4, 4a) zur Beheizung und die Signalverarbeitungseinheit (20) mit einer Temperaturregelung (16) ausgerüstet ist, welche die Organoberfläche an der Applikationsstelle des Sensors (1) auf eine der Körpertiefentemperatur entsprechende Temperatur beheizt und daß eine Anzeige für die Körpertiefentemperatur vorhanden ist.

Fig. 1

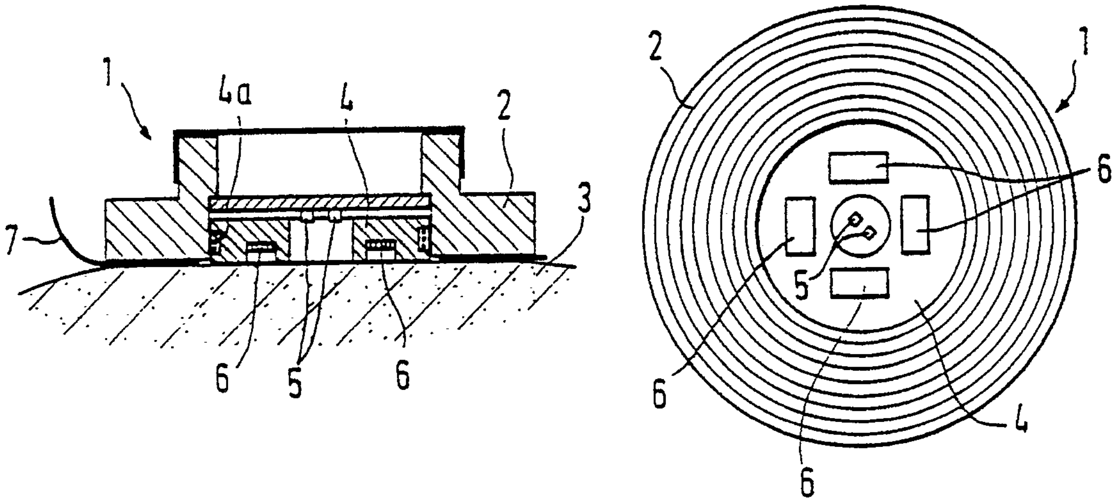


Fig. 2

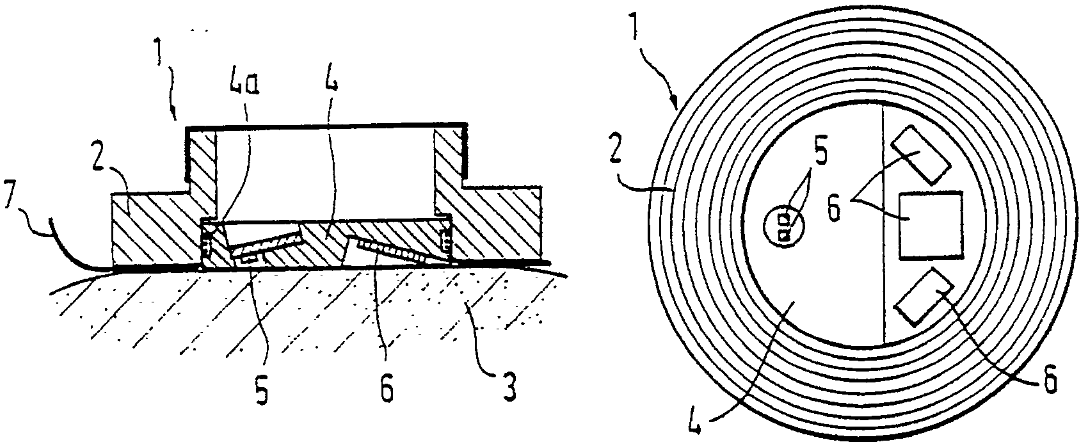

Fig. 3

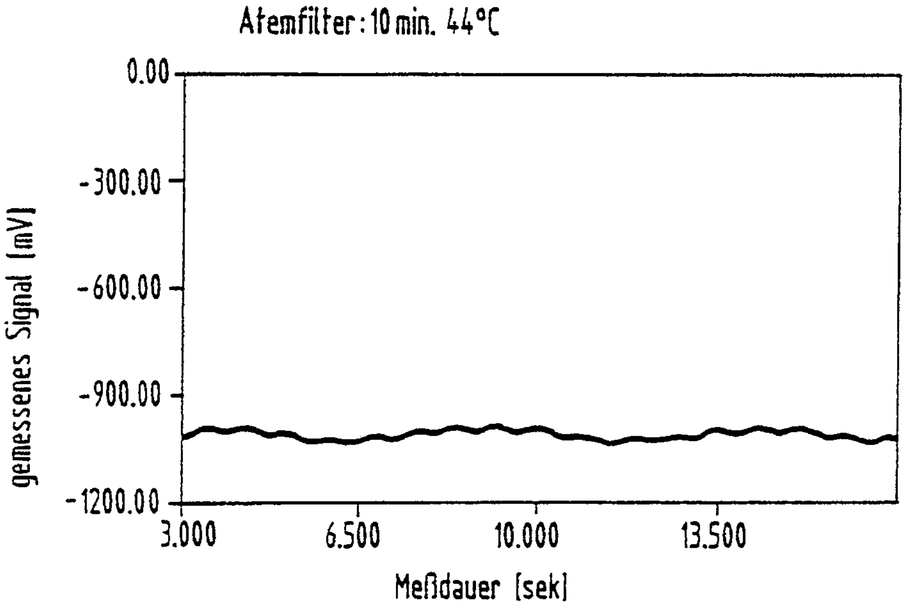
Atemfilter: 10 min. 44°C
0.00
-300.00
-600.00
-900.00
-1200.00
gemessenes Signal [mV]
3.000
6.500
10.000
13.500
Meßdauer [sek]

Fig. 4

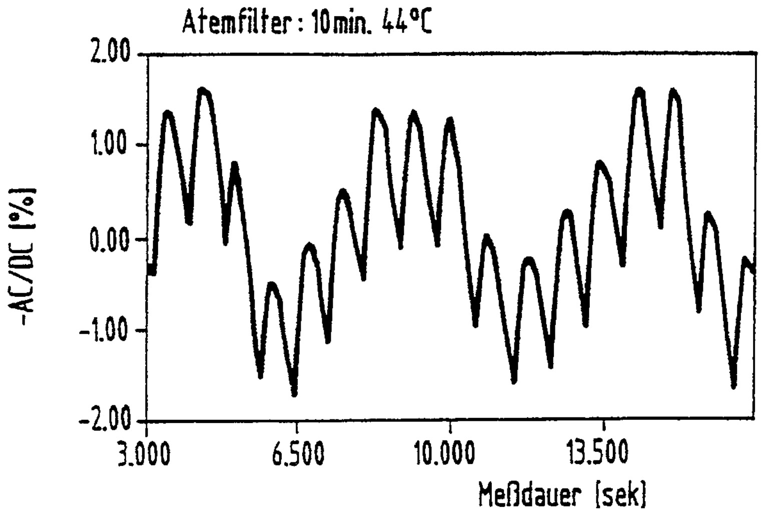


Fig. 5

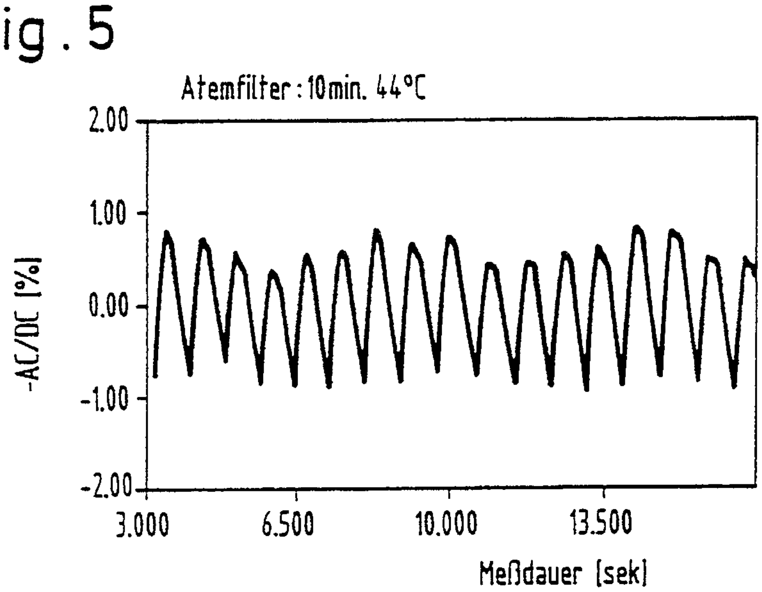


Fig. 6

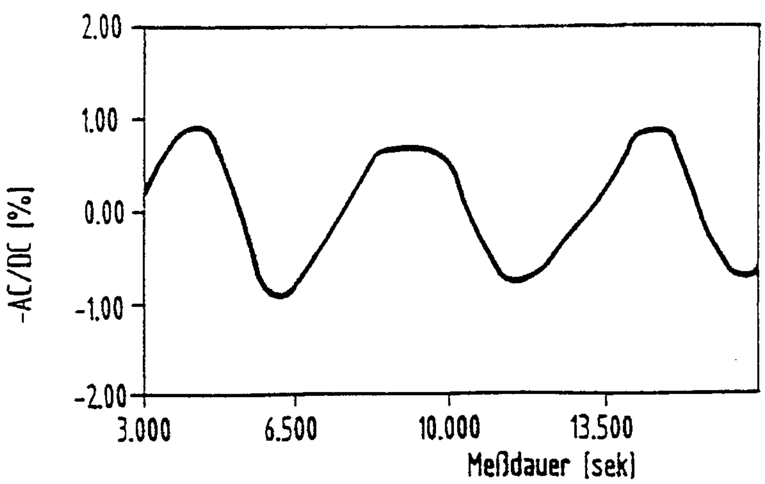

Fig. 7

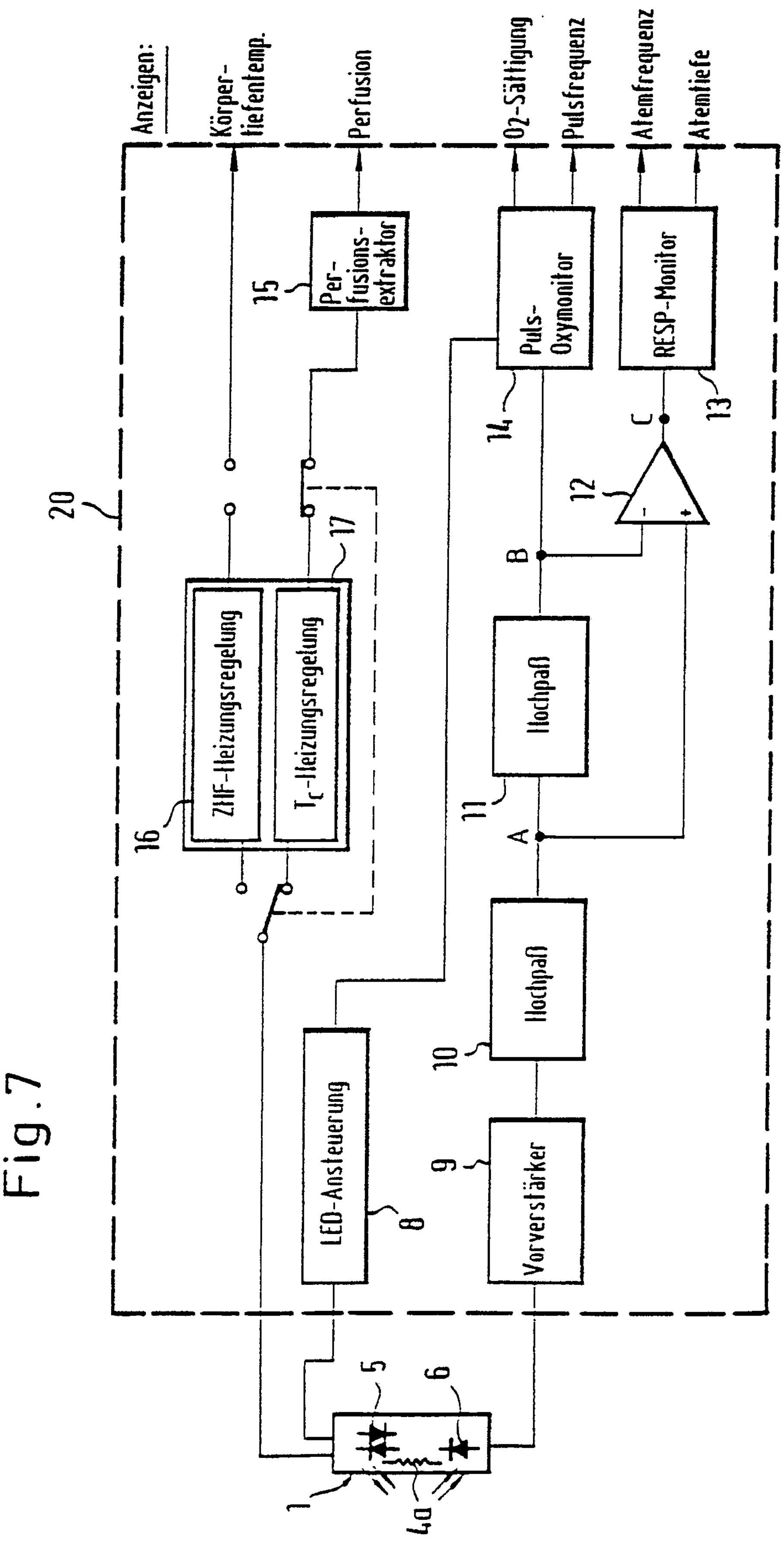
Anzeigen:
Körper-tiefentemp.
Perfusion
$O_2$-Sättigung
Pulsfrequenz
Atemfrequenz
Atemtiefe
20
Per-fusions-extraktor
15
Puls-Oxymonitor
14
RESP-Monitor
13
C
12
B
A
17
ZHF-Heizungsregelung
$T_c$-Heizungsregelung
16
Hochpaß
11
Hochpaß
10
LED-Ansteuerung
8
Vorverstärker
9
5
6
1
4a